# EUROPÄISCHE PATENTANMELDUNG

(11) **EP 4 616 879 A1**
(43) Veröffentlichungstag der Anmeldung: **17.09.2025**
(21) Anmeldenummer: 24163941.8
(22) Anmeldetag: 15.03.2024
(51) Int. Cl.: A61M 5/145, A61M 5/315

(54) **MEDIZINISCHER EINMALARTIKEL MEDIZINISCHE VORRICHTUNG**

(71) Anmelder: B. Braun Melsungen AG, 34212 Melsungen (DE)
(72) Erfinder: Kauba, Michael, 34277 Fuldabrück (DE); Vogel, Markus, 37191 Katlenburg-Lindau (DE); Sielemann, Martin, 34466 Wolfhagen (DE); Ostermoeller, Michael, 36251 Bad Hersfeld (DE)
(74) Vertreter: Tergau & Walkenhorst Intellectual Property GmbH

(57) **Zusammenfassung**

Medizinischer Einmalartikel (1) für eine medizinische Vorrichtung (2), insbesondere Spritzenpumpen, wobei der Einmalartikel (1) bevorzugt eine Spritze zur Verwendung in Spritzenpumpen ist, umfassend ein maschinenlesbares Erkennungsmerkmal (3), wobei das maschinenlesbare Erkennungsmerkmal (3) ein Merkmal des Einmalartikels (1) ist oder ein an einem vorbestimmten Teilabschnitt (11) des Einmalartikels (1) lösbar oder unlösbar, insbesondere werkseitig, angebracht ist.

## Beschreibung

### Technisches Gebiet

Die vorliegende Erfindung betrifft einen medizinischen Einmalartikel für eine medizinische Vorrichtung, insbesondere eine Spritze zur Verwendung in einer Spritzenpumpe, eine medizinische Vorrichtung, insbesondere einen Spritzenpumpe, zur automatisierten Verabreichung von Medikamenten an einen Patienten, ein Maschinenlesbares Erkennungsmerkmal für Einmalartikel in einer medizinischen Vorrichtung, ein System und ein Verfahren zur Erkennungsmerkmal abhängigen, insbesondere personalisierten, Medikamentenverabreichung.

### Hintergrund der Erfindung

Im Stand der Technik sind medizinische Vorrichtungen, insbesondere Spritzenpumpen bekannt, die dazu dienen in der Therapie eines Patienten eingesetzt zu werden und insbesondere Medikamente an den Patienten automatisiert zu verabreichen. Dabei werden medizinische Einmalartikel, wie beispielsweise Spritzen zur Verwendung in Spritzenpumpen, in die medizinische Vorrichtung eingebracht, die sodann das in dem Einmalartikel, beispielsweise einer Spritze zur Verwendung in Spritzenpumpen, enthaltene Medikament kontrolliert an den Patienten befördert. Dabei wird insbesondere eine in der medizinischen Vorrichtung vorhandene Antriebseinheit verwendet, die durch eine entsprechende Steuereinrichtung angesteuert werden kann.

Beispielsweise kann ein Medikament oder eine andere medizinische Lösung, wie beispielsweise Kochsalzlösung, in einer Spritze zur Verwendung in Spritzenpumpen bereitgestellt werden. Diese Zubehörartikel mit beinhaltendem Medikament können dann in die medizinische Vorrichtung eingebracht werden. Sodann kann der Aktuator, der die Spritze betätigt, so angesteuert werden, dass die gewünschte Menge an Lösung dem Patienten verabreicht wird. Dies wird beispielsweise dadurch erreicht, dass die Antriebseinheit und ggf. ein von dieser umfasster Antriebskopf, durch Vorschub eines Membrantellers, diesen auf eine Spritzenkolbenplatte drückt und so den Stößel/ Spritzenkolben in den Spritzenkolben drückt. Durch den entstehenden Druck wird die gewünschte Menge der Lösung kontrolliert aus der -Spritzenöffnung einer Spritze zur Verwendung in Spritzenpumpen entlassen.

Es sind eine Vielzahl solcher medizinischen Vorrichtungen, sowie zugehöriger Zubehörartikel im Stand der Technik bekannt.

Es besteht jedoch im Allgemeinen das Problem, dass im medizinischen Alltag und gegebenenfalls im Hausgebrauch das richtige Medikament, über den gewünschten Zugang, in der gewünschten Menge und Zeit an den Patienten gelangt, und das medizinische System, umfassend die medizinische Vorrichtung und den medizinischen Zubehörartikel so eingerichtet sein soll, dass eine Verwechslung oder anderweitige versehentliche Falschbedienung möglichst ausgeschlossen wird.

In der Praxis wählt zumeist klinisches Personal einen mit medizinischer Lösung und gegebenenfalls Medikament vorbestückten Zubehörartikel, oder bestückt diesen selbst mit einer Lösung und gegebenenfalls einem Medikament, und setzt sodann den bestückten Zubehörartikeln in die medizinische Vorrichtung ein. Gegebenenfalls kann an einer grafischen Benutzeroberfläche der medizinischen Vorrichtung der ausgewählte Zubehörartikel ausgewählt und bestätigt werden. Modernere Systeme verfügen über eine entsprechende Netzwerkschnittstelle und kommunizieren gegebenenfalls mit einer externen Steuereinheit.

Dies ist von besonderer Relevanz, wenn medizinische Systeme, beispielsweise im intensivmedizinischen Bereich, eine Vielzahl von medizinischen Vorrichtungen umfassen, um den Patienten zur selben Zeit eine Vielzahl von verschiedenen Medikamenten und Lösungen, gegebenenfalls über eine Vielzahl von Zugängen bereitzustellen. Dann können hierdurch eine Vielzahl von medizinischen Schläuchen den Patienten umgeben und die Sicherheit vor Fehlern beim Anschluss durch medizinisches Personal kann beeinträchtigt sein.

Wird das falsche Medikament verabreicht, oder eine falsche Förderleistung der medizinischen Vorrichtung gewählt, kann dies zu fatalen Folgen für den Patienten führen. Insbesondere kann die Gabe von Medikamenten in den falschen Patientenzugang unmittelbar hochkritisch sein, oder - insbesondere bei eingestellten geringen Förderraten - kann ein etwaiger vorhandener Alarm erst spät ausgelöst werden, der eine zu geringe Gabe an Medikament oder medizinischer Lösung detektieren würde. Auch die Vermischung von ggf. nicht miteinander kompatiblen Medikamenten und/oder Lösungen kann durch eine fehlende Übersicht über den Leitungsverlauf verschiedener Zubehörartikel und medizinischer Vorrichtungen vorkommen.

Gegenwärtig wird die Identifizierung der geeigneten Zubehörartikel von Hand durchgeführt und insbesondere durch eine visuelle und manuelle Verfolgung des Schlauchs von der medizinischen Vorrichtung hin zum Patientenzugang. Dabei sind im Stand der Technik verschiedene Lösungen bekannt, die insbesondere eine Markierung der Zubehörartikel vorsehen, um einer Verwechslung beim Bestücken der medizinischen Vorrichtung mit dem Zubehörartikel möglichst entgegenzuwirken.

Beispielsweise offenbart Dokument, WO 23280455 A1 einen medizinischen Schlauch oder eine medizinische Gleitschelle, zur Verwendung an oder in einem medizinischen Gerät, wobei der Einwegmedizinartikel mindestens eine integrierte Markierung als Identifikationsmerkmal umfasst.

Es bleibt dabei insbesondere auch mit Hinblick auf den Einsatz in einem Mehrkomponentensystem problematisch, Fehler beim Anschluss und in der Zuordnung von Zubehörartikel und medizinischer Vorrichtung zu vermeiden.

### Kurze Zusammenfassung der Erfindung

Es ist deshalb eine der Erfindung zu Grunde liegende Aufgabe, die oben genannten und weitere Nachteile zu überwinden. Dabei ist es eine weitere der Erfindung zu Grunde liegende Aufgabe, die Sicherheit beim bestimmungsgemäßen Anschluss von medizinischen Lösungen und ggf. Medikamente umfassenden Einmalartikeln, insbesondere von Spritze zur Verwendung in Spritzenpumpen, in eine medizinische Vorrichtung zu erhöhen und insbesondere sicherzustellen, dass der jeweilige Einmalartikel der bestimmungsgemäß richtigen medizinischen Vorrichtung zugeordnet wird. Darüber hinaus ist es eine der Erfindung zu Grunde liegende Aufgabe, die bestimmungsgemäße Einstellung der medizinischen Vorrichtung, insbesondere mit Hinblick auf die Abgabe von medizinischer Lösung und ggf. Medikament, das im Zubehörartikel umfasst ist, an den Patienten, zu erleichtern.

Diese und andere Probleme werden durch den Gegenstand der beigefügten unabhängigen Ansprüche gelöst.

### Detailbeschreibung der Erfindung

Die eingangs beschriebenen Aufgaben werden gelöst durch einen medizinischen Einmalartikel für eine medizinische Vorrichtung, insbesondere eine Spritze zur Verwendung in Spritzenpumpen, eine medizinische Vorrichtung, insbesondere einen Spritzenpumpe, zur automatisierten Verabreichung von Medikamenten an einen Patienten, ein Maschinenlesbares Erkennungsmerkmal für Einmalartikel in einer medizinischen Vorrichtung, ein System und ein Verfahren zur Erkennungsmerkmal abhängigen, insbesondere personalisierten, Medikamentenverabreichung gemäß den beigefügten unabhängigen Ansprüchen.

Bevorzugte Ausführungsformen können aus den abhängigen Ansprüchen sowie darüber hinaus aus der folgenden Beschreibung entnommen werden, insbesondere umfassend verschiedene Ausführungsformen, wie in den beigefügten Ansprüchen dargelegt und beschrieben.

Der Fachmann wird dabei verstehen, dass jede Ausführungsform, die in der folgenden Beschreibung beschrieben ist, vom Gegenstand der beigefügten Ansprüche erfasst und umfasst wird. Die Ausführungsformen, Merkmale und Kombinationen von Merkmalen, wie hier in Verbindung mit der Erfindung beschrieben, sowie die Kombination von Merkmalen, wie in den beigefügten Ansprüchen angegeben, aber auch jede Kombination von Merkmalen, wie in Verbindung mit den Ausführungsformen erwähnt und beschrieben, gelten als hier offenbart, mindestens jedoch als durch den Fachmann ableitbar.

Insbesondere kann jede Funktion und jede Kombination von Funktionen in den vorliegenden Ausführungsformen beispielsweise in einer unterschiedlichen Kombination beansprucht werden, insbesondere in einer unterschiedlichen Anspruchskategorie, nicht zuletzt, weil der Fachmann erkennen wird, dass jede einzelne Kombination der hier genannten Funktionen dazu geeignet ist, zur Lösung der zugrunde liegenden Aufgabe beizutragen.

Des Weiteren können jede Funktion und jede Kombination von Funktionen in den Ansprüchen und in der untenstehenden Beschreibung unabhängig von dem jeweils beanspruchten Gegenstand verwendet und beansprucht werden, unabhängig von Anspruchsabhängigkeiten und Rückbezügen, sowie unabhängig von der Anspruchskategorie, in der die Funktion beansprucht wird. Zum Beispiel kann in einer willkürlichen Kombination, die aus einem oder mehreren Ansprüchen ausgewählt ist, eine oder mehrere der nachstehend beschriebenen Ausführungsformen und/oder aus den beigefügten Abbildungen vorgestellten Ausführungsformen in Betracht gezogen werden.

Im Rahmen der vorliegenden Anmeldung beziehen sich Begriffe wie "seitlich", "hinten", "vorne", "oben", "unten", "gegenüber", "innen", "außen" oder ähnliche, wie hier verwendet, die die Position eines ersten Objekts relativ zu einem anderen Objekt beschreiben, bevorzugt auf die relative Position eines jeweiligen Teils oder Objekts in Bezug auf seine Position, wenn es vollständig für den vorgesehenen Gebrauch montiert ist. In anderen Worten, werden diese Begriffe bevorzugt in Bezug auf einen erfindungsgemäßen Einmalartikel oder eine erfindungsgemäße medizinische Vorrichtung mit Hinblick auf die relative Position des jeweiligen Teils oder Objekts in Bezug auf seine Position verwandt, wenn der erfindungsgemäße Einmalartikel vollständig für den vorgesehenen Gebrauch in der erfindungsgemäße medizinische Vorrichtung montiert ist.

Nach einem ersten Aspekt der vorliegenden Erfindung, betrifft die Erfindung einen medizinischen Einmalartikel für eine medizinische Vorrichtung, insbesondere Spritzenpumpen, wobei der Einmalartikel bevorzugt eine Spritze zur Verwendung in Spritzenpumpen ist. Ein erfindungsgemäßer Einmalartikel umfasst ein Erkennungsmerkmal, wobei das maschinenlesbare Erkennungsmerkmal ein Merkmal des Einmalartikels ist oder ein an einem vorbestimmten Teilabschnitt des Einmalartikels lösbar oder unlösbar, insbesondere werkseitig, angebracht ist. Eine medizinische Vorrichtung gemäß der vorliegenden Erfindung ist bevorzugt ein Spritzenpumpe, bzw. eine Spritzenpumpepumpe. Ein medizinischer Einmalartikel gemäß der vorliegenden Erfindung ist bevorzugt eine Spritze zur Verwendung in Spritzenpumpen.

Der Begriff "Erkennungsmerkmal", wie hierin verwendet, bezeichnet bevorzugt jedwedes Merkmal, das geeignet ist, einen bestimmungsgemäßen Einmalartikel gemäß der vorliegenden Erfindung von anderen zu unterscheiden oder zu identifizieren. In anderen Worten ist ein maschinenlesbares Erkennungsmerkmal ein charakteristisches Element, eine Eigenschaft oder eine Kennzeichnung, die dazu dient, einen bestimmungsgemäßen Einmalartikel gemäß der vorliegenden Erfindung von anderen zu unterscheiden oder zu identifizieren.

In verschiedenen Kontexten kann ein maschinenlesbares Erkennungsmerkmal durch visuelle, technologische, physikalische oder andere Merkmale dargestellt werden. Dieses Merkmal ermöglicht die eindeutige Identifizierung oder Unterscheidung im Rahmen eines bestimmten Systems, einer Anwendung oder einer Aufgabe. Beispiele für Erkennungsmerkmale sind Farbcodes, Barcodes, QR-Codes, biometrische Merkmale, RFID-Tags oder andere charakteristische Eigenschaften, die zur Identifizierung dienen, wie hierin beschrieben.

Insbesondere ein RFID-Tag hat den Vorteil, dass dieses über die reine Identifikation des Einmalartikels hinausgehende, komplexere Daten, speichern und tragen kann. Beispielsweise kann ein RFID-Tag Informationen enthalten, die Informationen zum Patienten und/oder Anwendungen umfassen. Zusätzlich bietet sich die Möglichkeit, Anwendungsinformationen auf den RFID-Tag zurückzuschreiben. So kann beispielsweise auch gesichert werden, dass das exakte Ablaufdatum des Artikels nicht überschritten wird.

Der Einmalartikel kann ein oder mehrteilig gefertigt sein. Dabei ist das maschinenlesbare Erkennungsmerkmal dafür vorgesehen, den Einmalartikel so zu markieren, dass dieser von anderen Einmalartikeln unterschieden bzw. anhand des Erkennungsmerkmals identifiziert werden kann. Dies geschieht insbesondere anhand der Markierung des Einmalartikels mittels des Erkennungsmerkmals an einem vorbestimmten Teilabschnitt des Einmalartikels. Erfindungsgemäß kann das maschinenlesbare Erkennungsmerkmal lösbar oder unlösbar, insbesondere werkseitig, an den Einmalartikel angebracht sein. Das maschinenlesbare Erkennungsmerkmal kann zusätzlich oder alternativ auch ein Merkmal des Einmalartikels selbst sein, bspw. die Farbe der Spritze. Ein Erkennungsmerkmal das "ein an einem vorbestimmten Teilabschnitt des Einmalartikels lösbar oder unlösbar, insbesondere werkseitig, angebracht ist" kann dabei bevorzugt zusätzlich oder alternativ auch als Merkmal verstanden werden, das als integrales Merkmal des Teilabschnitts oder Teils des Einmalartikels ausgeprägt ist, bspw. die Farbe des Spritzenstößels.

Möglich ist es bspw. bereits werksseitig einen Einmalartikel durch ein permanentes Anbringen des Erkennungsmerkmals identifizierbar zu machen. So kann ein solches Merkmal bauseitig vorgesehen sein, zum Beispiel angeformt sein oder in einer Gussform als integrales Teil, geformt sein. Alternativ kann das Erkennungsmerkmal aufgedruckt sein. Alternativ oder zusätzlich kann der Einmalartikel manuell, bspw. durch Klinikpersonal, mit einem maschinenlesbares Erkennungsmerkmal lösbar oder unlösbar versehen werden. Beispielsweise, kann durch Aufklipsen, Aufstecken, oder durch Anbringen eines Merkmals in Form oder mit Hilfe eines Befestigungsmittels, bspw. einer Gummitülle, die gewünschte Markierung und Individualisierung erreicht werden. Besonders das nachträgliche Anbringen eines Erkennungsmerkmals vor Anwendung, insbesondere werksseitig, erlaubt eine gezieltes und Benutzer-bestimmtes Individualisieren. Insbesondere ein Spritzenkolben oder eine Spritzenkolbenplatte kann jedoch auch bedruckt und bereits entsprechend werkseitig mit einem Erkennungsmerkmal versehen sein. Alternativ kann insbesondere an eine Spritzenkolbenplatte auch ein Erkennungsmerkmal geklippt oder aufgesteckt werden.

Die vorliegende Erfindung und der medizinische Einmalartikel, insbesondere in Form einer Spritze zur Verwendung in Spritzenpumpen, mit dem Erkennungsmerkmal des Einmalartikels erlaubt besonders vorteilhaft Therapiesets und die Medikation vor allem Anwendung- und/oder Patientenbezogen zuordenbar zu machen, insbesondere vorzukonfigurieren. Dies eröffnet insbesondere Vorteile in der Erhöhung der Sicherheit und erlaubt ggf. weniger geschultes Personal, bspw. Privatpersonen im Home Care Bereich, eine sichere Anwendung des Einmalartikels in der medizinischen Vorrichtung.

Das maschinenlesbare Erkennungsmerkmal, insbesondere, der vorbestimmte Teilabschnitt, ist bevorzugt so ausgewählt, dass dieses von einer medizinischen Vorrichtung, bevorzugt in einem eingesetzten Zustand, eingelesen werden kann. Der vorbestimmte Teilabschnitt ist aus der Gruppe umfassend Spritzenzylinderflügel, Stößel und/oder Spritzenkolben und/oder Spritzenkolbenplatte, bevorzugt einer Spritze zur Verwendung in Spritzenpumpen ausgewählt.

In einer vorteilhaften Ausführungsform des erfindungsgemäßen Einmalartikels ist der vorbestimmte Teilabschnitt ein, Teil, das einem Antriebskopf, insbesondere einem Membranteller, einer medizinischen Vorrichtung zugewandt ist, insbesondere eine Spritzenkolbenplatte. Diese gezielte Ausrichtung erleichtert die präzise Identifikation und Anwendung des Einmalartikels durch die medizinische Vorrichtung an einer besonders zugänglichen Stelle, da der Antriebskopf und insbesondere der Membranteller, bestimmungsgemäß mit dem Einmalartikel, nämlich der Spritzenkolbenplatte in Kontakt kommen, um den Spritzenkolben in den Spritzenzylinder zu drücken.

Der Begriff "in einem eingesetzten Zustand", wie hierin verwendet bezeichnet dabei bevorzugt die korrekte Lage des Einmalartikels in der medizinischen Vorrichtung für den bestimmungsgemäßen Gebrauch, also wenn bspw. die Spritze zur Verwendung in Spritzenpumpen korrekt in den medizinischen Spritzenpumpe eingebracht und gehaltert ist.

In einer weiteren vorteilhaften Ausführungsform des erfindungsgemäßen Einmalartikels ist das maschinenlesbare Erkennungsmerkmal ausgewählt aus
- einer formgebundenen Kodierung, insbesondere einer 3D Struktur,
- einer elektronisch-physikalischen Messgröße, insbesondere einer Leitfähigkeit, einer Magnetisierung und/oder einer Drahtloswelle, insbesondere einem RFID-Tag, und/oder
- einem optischen Code, insbesondere einer Farbe und/oder einem Muster, einem Barcode oder QR-Code.

Besonders vorteilhaft ist das Erkennungsmerkmal als eine einer formgebundenen Kodierung, insbesondere einer 3D Struktur, vorgesehen und ausgestaltet. Diese formgebundene Kodierung kann durch verschiedene Merkmale wie Anformung, Relief, Riffelung und ähnliche charakterisiert sein. Die Anformung bezieht sich bspw. auf die spezifische Gestaltung oder Kontur eines Bereichs auf dem Einmalartikel, der als Erkennungsmerkmal dient. Das Relief beschreibt bspw. Erhebungen oder Vertiefungen auf der Oberfläche des Einmalartikels, die eine bestimmte Musterung oder Textur bilden. Riffelung bspw. bezieht sich auf regelmäßige Erhebungen, oft in Form von Rillen oder Wellen, die über die Oberfläche verteilt sind.

Durch die Integration dieser formgebundenen Kodierung, insbesondere als dreidimensionalen Struktur, in den vorbestimmten Teilabschnitt des Einmalartikels wird eine eindeutige und taktil erfassbare Kodierung geschaffen. Dies ermöglicht eine zuverlässige Identifikation des Einmalartikels durch Berührung oder taktile Erfassung, was insbesondere in Umgebungen, in denen visuelle Identifikationsmethoden eingeschränkt sind, von Vorteil ist.

Zusätzlich oder alternativ ist das Erkennungsmerkmal als eine elektronisch-physikalischen Messgröße, insbesondere einer Leitfähigkeit, einer Magnetisierung und/oder einer Drahtloswelle, insbesondere einem RFID-Tag, ausgestaltet. Die Erkennungsmerkmal als eine elektronisch-physikalischen Messgröße können zusätzlich oder alternativ ein "Miniatur Shield" umfassen. Ein Miniatur Shield bezeichnet bevorzugt eine Erweiterungsplatine oder -komponente, die insbesondere auf ein Mikrocontroller-Board angebracht sein kann und/oder aufgesteckt wird. Der Begriff "Miniatur Shield" soll dabei bevorzugt bedeuten, dass es sich um eine kompakte oder kleine Version eines solchen Erweiterungsmoduls handelt. Dies kann mit dem geringen Platz oder Gewichts-anforderungen zur Realisierung der Erfindung vorteilhaft sein. Ein Miniatur Shield kann erfindungsgemäß einem Teil der Sensoranordnung, insbesondere Sensoren, aber auch Kommunikationsmodule oder andere Komponenten enthalten, um die Funktionalität des Mikrocontrollers zu erweitern.

Die Leitfähigkeit bezieht sich dabei bevorzugt auf die Fähigkeit des Einmalartikels, elektrischen Strom zu leiten, was als messbare physikalische Eigenschaft genutzt werden kann. Die Magnetisierung bezieht sich bspw. auf die Ausrichtung von magnetischen Momenten im Material des Einmalartikels, die wiederum eine charakteristische Messgröße darstellen können. Drahtloswellen, insbesondere RFID-Tags, ermöglichen bevorzugt die drahtlose Identifikation und Kommunikation durch elektromagnetische Wellen. Durch Integration derartiger elektronisch-physikalischen Messgrößen in den vorbestimmten Teilabschnitt des Einmalartikels wird vorteilhaft eine automatisierte und präzise Identifikation ermöglicht. Dies ist besonders nützlich für schnelle und berührungslose Identifikationsprozesse in verschiedenen Anwendungen, wie beispielsweise in der Medizintechnik.

Zusätzlich oder alternativ ist das maschinenlesbare Erkennungsmerkmal als ein optischer Code, insbesondere eine Farbe und/oder ein Muster, einen Barcode oder QR-Code, ausgestaltet. Die Farbe kann sowohl schwarz-weiß als auch verschiedene Farben umfassen. Die Farbe repräsentiert hierbei eine visuell wahrnehmbare Eigenschaft des Einmalartikels und/oder vorbestimmten Teilabschnitts des Einmalartikels. Ein Muster kann zusätzliche visuelle Elemente beinhalten, die als charakteristisch für die Identifikation dienen. Der Barcode und QR-Code stellen spezifische optische Codes dar, die durch Scannen oder fotografische Erfassung maschinenlesbar sind und Informationen über den Einmalartikel enthalten. Durch diese Integration optischer Codes wird eine effiziente und präzise visuelle Identifikation des Einmalartikels ermöglicht, insbesondere in Anwendungen, die auf optischer Erkennung basieren, wie beispielsweise bei der Verwendung von Scannern oder Kameras. Insbesondere kann ein illuminierter RFID-Reader als Erkennungsmerkmal verwendet werden. Viele dieser Reader-Module verfügen über einen Kontrollring, der zur Signalisierung genutzt werden kann. Dies trägt dazu bei, die Anwendungssicherheit zusätzlich zu erhöhen. Die Beleuchtung während des Lesevorgangs verbessert die Lesbarkeit (Kontrast) und stellt sicher, dass die Identifikation zuverlässig erfolgt. Dieses Konzept kann jedoch generell bei jeder Erkennungsmethode angewendet werden, um die Anwenderführung zu optimieren und zu sichern. Es trägt dazu bei, eine intuitive Benutzerführung zu gewährleisten und die Identifikation von Einmalartikeln effizient und sicher zu gestalten. So kann ein derart beleuchteter Ring zusammen mit oder unabhängig von einem RFID-Tag verwendet werden.

In einem zweiten Aspekt der vorliegenden Erfindung, betrifft die Erfindung eine Medizinische Vorrichtung, insbesondere Spritzenpumpe, zur automatisierten Verabreichung von Medikamenten an einen Patienten. Eine erfindungsgemäße medizinische Vorrichtung umfasst
- eine Erkennungseinrichtung zur Identifikation eines zuordenbaren, klassifizierbaren und/oder personalisierbaren Einmalartikels, umfassend eine Sensoranordnung, die dazu eingerichtet ist, das maschinenlesbare Erkennungsmerkmal des Einmalartikels zu erfassen;
- eine Steuereinrichtung, die mit der Sensoranordnung verbunden ist und auf Basis der erfassten Informationen das maschinenlesbare Erkennungsmerkmal des Einmalartikels identifiziert;
- eine Antriebseinheit, die durch die Steuereinrichtung gesteuert wird, um das Medikament gemäß den erkannten Informationen des Einmalartikels automatisiert an den Patienten zu verabreichen,
wobei der Einmalartikel, bevorzugt ein Einmalartikel nach einem der vorhergehenden Ansprüche ist.

Eine Erkennungseinrichtung zur Identifikation eines zuordenbaren, klassifizierbaren und/oder personalisierbaren Einmalartikels umfasst eine Sensoranordnung, die speziell darauf ausgerichtet ist, das maschinenlesbare Erkennungsmerkmal des Einmalartikels zu erfassen. Diese Sensoranordnung kann verschiedene Technologien und Sensoren umfassen, die darauf abzielen, das definierte Erkennungsmerkmal des Einmalartikels präzise zu registrieren. Eine Steuereinrichtung, die mit der Sensoranordnung verbunden ist, übernimmt die Verarbeitung der erfassten Informationen, um das maschinenlesbare Erkennungsmerkmal des Einmalartikels zu identifizieren. Die Steuereinrichtung fungiert bevorzugt als zentrales Verarbeitungselement und nutzt die von der Sensoranordnung bereitgestellten Daten, um den Einmalartikel eindeutig zu identifizieren. Die Verbindung zwischen der Sensoranordnung und der Steuereinrichtung ermöglicht einen nahtlosen Informationsaustausch, wodurch die erfassten Daten effizient und präzise analysiert werden können. Dabei ist bevorzugt zwischen Sensoranordung und Steuereinrichtung eine Verarbeitungseinrichtung vorgesehen, die die mit dem Sensor erfassten Informationen verarbeitet, und ggf. dazu gehörige Daten aus einer Speichereinheit abgerufen werden.

Auf Basis dieser Analyse identifiziert die Steuereinrichtung das spezifische Erkennungsmerkmal, das dem zuordenbaren, klassifizierbaren und/oder personalisierbaren Einmalartikel zugeordnet ist. Diese Identifikation bildet die Grundlage für die weiteren Schritte in der Anwendung, sei es in der Medikamentenverabreichung oder einem anderen Kontext, in dem der Einmalartikel eingesetzt wird. Eine Antriebseinheit wird durch die Steuereinrichtung gesteuert, um das Medikament gemäß den erkannten Informationen des Einmalartikels automatisiert an den Patienten zu verabreichen. Die Steuereinrichtung orchestriert demnach die Funktionen der Antriebseinheit basierend auf den identifizierten Informationen des Einmalartikels. Die Antriebseinheit kann verschiedene mechanische oder elektronische Mechanismen umfassen, die dazu dienen, das Medikament gemäß den spezifischen Anforderungen und Dosierungen an den Patienten zu liefern. Insbesondere ist im Stand der Technik die Führung des Spritzenkolbens einer Spritze zur Verwendung in Spritzenpumpen innerhalb des Spritzenzylinders mittels eines durch einen Antriebskopf bewegten Membrantellers bekannt. Durch die Integration mit der Steuereinrichtung wird die Verabreichung des Medikaments automatisiert und präzise gesteuert, wodurch die Sicherheit und Effektivität der medizinischen Behandlung verbessert wird.

In einer vorteilhaften Ausführungsform der erfindungsgemäßen Medizinischen Vorrichtung, umfasst diese eine Aktivierungseinrichtung zur Aktivierung und Deaktivierung der Erkennungseinrichtung. Die ist vorteilhaft, in dem die Erkennungseinrichtung nach Bedarf aktiviert oder deaktiviert werden kann. Mit anderen Worten kann die Erkennung deaktiviert werden, wenn sie nicht benötigt wird. Dies kann bspw. dazu beitragen, Energie zu sparen und eine Batterielaufzeit zu verlängern. Bei Bedarf kann die Aktivierungseinrichtung die Erkennungseinrichtung aktivieren, um die Identifikation von Einmalartikeln wieder zu ermöglichen. Dieser Ansatz verbessert die Energieeffizienz der medizinischen Vorrichtung und gewährleistet, dass die Erkennungseinrichtung nur dann in Betrieb ist, wenn sie tatsächlich benötigt wird, was insbesondere in batteriebetriebenen Geräten einen praktischen Vorteil darstellen kann. Zum Aktivieren und/oder Deaktivieren kann die medizinische Vorrichtung, insbesondere in einem Display, ein entsprechendes Betätigungselement aufweisen.

In einer weiteren vorteilhaften Ausführungsform der erfindungsgemäßen Medizinischen Vorrichtung umfasst die Vorrichtung eine Kommunikationsschnittstelle zur Übermittlung der erfassten Informationen an externe Steuergeräte oder Netzwerke. Ein bedeutender Vorteil dieser Konfiguration besteht darin, dass die erfassten Informationen über die Kommunikationsschnittstelle mit externen Geräten oder Netzwerken geteilt werden können. Dies ermöglicht eine nahtlose Integration der Medizinischen Vorrichtung in umfassendere medizinische Systeme oder Datenplattformen. Externe Steuergeräte können die erhaltenen Informationen nutzen, um zusätzliche Funktionen zu steuern oder um übergeordnete Entscheidungen zu treffen. Die Übermittlung an Netzwerke ermöglicht eine Fernüberwachung und - steuerung der Medizinischen Vorrichtung, was insbesondere in umfangreichen medizinischen Infrastrukturen von Vorteil ist. Besonders vorteilhaft ist damit die Einbindung der erfindungsgemäßen Vorrichtung in ein erfindungsgemäßes System ermöglicht, so dass bspw. eine Cloud-basierte Anwendungsdatenbank, die bevorzugt Informationen zu Komponenten des Systems enthält, darunter Angaben zur Pumpe, Patienten-ID, Behandlungsschemata und Medikamentendetails, durch die medizinische Vorrichtung, mittels der Kommunikationsschnittstelle angesprochen werden kann und erfasste Informationen übermitteln bzw. mit Informationen der Cloud-basierte Anwendungsdatenbank abgeglichen werden können.

In einer weiteren vorteilhaften Ausführungsform der erfindungsgemäßen medizinischen Vorrichtung umfasst die Antriebseinheit, insbesondere ein Antriebskopf oder Membranteller der Antriebseinheit, die Erkennungseinrichtung.

Bevorzugt ist die Erkennungseinrichtung als Teil der Antriebseinheit, insbesondere einem Antriebskopf der Antriebseinheit, ausgebildet und/oder an der Antriebseinheit, insbesondere einem Antriebskopf der Antriebseinheit, angebracht.

Die Antriebseinheit, insbesondere ein Antriebskopf oder Membranteller, umfasst dabei bevorzugt die Erkennungseinrichtung umfasst und/oder ebenfalls bevorzugt ist die Erkennungseinrichtung als Teil der Antriebseinheit, insbesondere eines Antriebskopfs der Antriebseinheit, ausgebildet und/oder ebenfalls bevorzugt ist die Erkennungseinrichtung an der Antriebseinheit, insbesondere einem Antriebskopf der Antriebseinheit, angebracht.

Diese Anordnung bietet den Vorteil, dass die Erkennungseinrichtung unmittelbar in die Antriebseinheit integriert ist. Dadurch wird eine kompakte Bauweise und eine verbesserte Funktionalität erzielt. Die Erkennungseinrichtung kann somit effizient mit dem Antriebsmechanismus zusammenarbeiten und präzise Informationen über den zuordenbaren, klassifizierbaren und/oder personalisierbaren Einmalartikel erfassen. Dies ist insbesondere der Fall, wenn Bauteile des medizinischen Einmalartikels, bspw. eine Spritzenkolbenplatte, das Erkennungsmerkmal tragen, das dann, einem Bauteil der Antriebseinheit bestimmungsgemäß nahekommt, oder in Kontakt mit diesem tritt, z.B. mit einem Membranteller. Dies trägt zu einer sicheren und zuverlässigen Erkennung des Erkennungsmerkmals durch die Erkennungseinrichtung bei, indem eine direkte Verbindung zwischen der Antriebseinheit mit Erkennungseinrichtung und dem Erkennungsmerkmal an einem durch die Antriebseinheit angetriebenen Bauteil des Einmalartikels gewährleistet wird.

In einer weiteren vorteilhaften Ausführungsform der erfindungsgemäßen medizinischen Vorrichtung weist die medizinische Vorrichtung einen Haltemechanismus, insbesondere Klemm-Mechanismus, zur Halterung des Einwegartikels in der medizinischen Vorrichtung auf. Der Haltemechanismus umfasst dabei die Erkennungseinrichtung. Bevorzugt ist die Erkennungseinrichtung an einem Haltemechanismus, insbesondere Klemm-Mechanismus, zur Halterung des Einwegartikels in der medizinischen Vorrichtung angebracht und/oder als Teil eines Haltemechanismus, insbesondere Klemm-Mechanismus, zur Halterung des Einwegartikels in der medizinischen Vorrichtung ausgebildet. Dies erlaubt beispielsweise, die Erkennung eines Erkennungsmerkmals, das an einem für die Halterung vorgesehenen Bauteils des Einmalartikels vorgesehen ist. So kann zum Beispiel die Erkennung eines am Spritzenzylinderflügel angebrachten Erkennungsmerkmals durch eine Erkennungseinrichtung an einem Haltemechanismus, insbesondere Klemm-Mechanismus, zur Halterung des Einwegartikels in der medizinischen Vorrichtung erreicht werden.

In einer weiteren vorteilhaften Ausführungsform der erfindungsgemäßen Medizinischen Vorrichtung weist die Antriebseinheit, insbesondere ein Antriebskopf oder Membranteller, eine Sensorsignalöffnung auf. Durch eine derartige Sensorsignalöffnung können die Detektionssignale der Sensoranordnung zum Erkennungsmerkmal gelangen. Beispielsweise kann erreicht werden, dass die Sensorsignale von der Sensoranordnung das maschinenlesbare Erkennungsmerkmal erfassen können. Insbesondere kann die Sensorsignalöffnung durch ein sensorsignalgängiges, insbesondere durchsichtiges Material, durch ein integriertes Fenster, eine optische Linse oder eine Materialauslassung und/oder Materialaussparung gebildet sein. Ist eine solche Sensorsignalöffnung bspw. in einem Membranteller angeordnet, kann zum Beispiel besonders vorteilhaft die Messung bspw. durch den Membranteller vorgenommen werden und die Sensoranordnung an einer anderen Stelle, insbesondere hinter dem Membranteller im Antriebskopf vorgesehen sein. Eine integrierte optische Linse kann zudem das Signal bündeln oder verstärken.

In einer weiteren vorteilhaften Ausführungsform der erfindungsgemäßen Medizinischen Vorrichtung umfasst die Erkennungseinrichtung einen mit der Sensoranordnung verbundenen, bevorzugt flexibel geformten, optischen Leiter, bevorzugt Lichtleiter. Dies hat den Vorteil, dass der optische Leiter, bevorzugt Lichtleiter, einerseits eine präzisere und flexiblere Erfassung des maschinenlesbaren Erkennungsmerkmals ermöglicht und andererseits Licht aus einer von der Sensoranordnung umfassten Lichtquelle leiten kann, um das Erkennungsmerkmal ggf. auszuleuchten und besser detektierbar zu machen. Durch die Flexibilität des optischen Leiters, bzw. Lichtleiters, kann die Sensoranordnung effizient an unterschiedliche Geometrien und Formen des zuordenbaren, klassifizierbaren und/oder personalisierbaren Einmalartikels und innerhalb der medizinischen Vorrichtung angepasst und angeordnet werden. Dies trägt dazu bei, dass die Erkennungseinrichtung vielseitig einsetzbar ist und eine zuverlässige Identifikation des Einmalartikels gewährleistet wird. Der flexible optische Leiter, insbesondere Lichtleiter, ermöglicht zudem eine optimale Lichtführung, was die Genauigkeit der Erfassung weiter verbessert. Die Informationsführung durch einen solchen flexiblen optischen Leiter kann insbesondere bei der Verwendung eines optischen Codes, insbesondere einer Farbe und/oder einem Muster, einem Barcode oder QR-Code, vorteilhaft sein. Besonders vorteilhaft ist die Ausgestaltung eines Membrantellers mit lichtdurchlässigem Material, wobei ein entsprechender Sensor der Sensoranordnung direkt an den Membranteller angekoppelt oder indirekt angekoppelt werden kann, und wobei dann das Messsignal durch einen optischen Leiter, z.B. einen Lichtleiter oder ähnliches, geführt werden kann. Weiterhin kann der Membranteller eine optische Linse oder ein optisches Fenster aufweisen. Auch in dieser Ausführungsform kann ein entsprechender Sensor der Sensoranordnung direkt an den Membranteller angekoppelt oder indirekt angekoppelt werden, und wobei dann das Messsignal durch einen optischen Leiter, zum Beispiel einen Lichtleiter oder ähnliches, geführt werden kann.

In einer weiteren vorteilhaften Ausführungsform der erfindungsgemäßen medizinischen Vorrichtung ist die Erkennungseinrichtung zur zentrischen Erfassung des Erkennungsmerkmals, insbesondere eines Erkennungsmerkmals auf einer Spritzenkolbenplatte, eingerichtet. Die präzise Ausrichtung der Sensoranordnung, so dass ein auf einer Spritzenkolbenplatte bereitgestelltes Erkennungsmerkmal zentriert, erfasst werden kann, kann vorteilhaft bei der Erfassung der durch das Erkennungsmerkmal bereitgestellten Informationen und des Erkennungsmerkmals selbst sein. Mit anderen Worten ist die Sensoranordnung so eingerichtet und ausgerichtet, dass sie ein bereitgestelltes Erkennungsmerkmal optimal erfassen kann.

In einer weiteren vorteilhaften Ausführungsform der erfindungsgemäßen Medizinischen Vorrichtung umfasst die Vorrichtung und insbesondere die Erkennungseinrichtung eine Verschattungseinrichtung zur wenigstens partiellen Verschattung des Erkennungsmerkmals. Dabei ist es bevorzugt, dass die Verschattungseinrichtung als Teil eines Haltemechanismus, insbesondere Klemm-Mechanismus, zur Halterung des Einwegartikels in der medizinischen Vorrichtung ausgebildet ist. Insbesondere in Ausführungsformen, bei denen als Erkennungsmerkmal ein optischer Code, insbesondere einer Farbe und/oder einem Muster, einem Barcode oder QR-Code, verwendet wird, kann es vorteilhaft sein, den Bereich des Erkennungsmerkmals zu beschatten und/oder zu verdunkeln und ggf. mit einer Lichtquelle auszuleuchten. Somit kann eine Einwirkung von Umgebungslicht vermindert werden, und die Ausleuchtung ggf. durch eine System-eigene Lichtquelle optimal gestaltet werden. Die kann insbesondere durch die Anbringung einer Verschattungseinrichtung an vorhandenen Bauteilen einer medizinischen Vorrichtung besonders vorteilhaft bereitgestellt werden. Beispielsweise kann in einem Spritzenpumpe für eine Spritze zur Verwendung in Spritzenpumpen ein umlaufender Spritzenkolben-Greifmechanismus ausgestaltet sein, um als Verschattung modifiziert und genutzt werden zu können.

In einer weiteren vorteilhaften Ausführungsform der erfindungsgemäßen Medizinischen Vorrichtung ist die Sensoranordnung geeignet das maschinenlesbare Erkennungsmerkmal zu detektieren. Eine solche Sensoranordnung kann insbesondere ausgewählt sein aus einer Sensoranordnung zur Detektion einer formgebundenen Kodierung, insbesondere einer 3D Struktur. Dies kann beispielsweise einen resistiven Touchsensor und/oder eine Abtastmechanik umfassen.

Eine solche Sensoranordnung kann insbesondere auch ausgewählt sein aus einer Sensoranordnung zur Detektion einer elektronisch-physikalischen Messgröße, insbesondere einer Leitfähigkeit, einer Magnetisierung und/oder einer Drahtloswelle, insbesondere einem RFID-Tag. Hier können insbesondere Leitfähigkeitssensoren, zum Messen des elektrischen Widerstands oder der Leitfähigkeit; Magnetfeldsensoren, zum Detektieren von Veränderungen im Magnetfeld, die auf eine Magnetisierung hindeuten können; oder Hall-Effekt-Sensoren, zum Erfassen von Änderungen im Magnetfeld basierend auf dem Hall-Effekt; oder RFID-Sensoren, zum Lesen und Interpretieren der Daten, die von RFID-Tags über Funkwellen gesendet werden, eingesetzt werden.

Eine solche Sensoranordnung kann insbesondere ausgewählt sein aus einer Sensoranordnung zur Detektion einem optischen Code, insbesondere einer Farbe und/oder einem Muster, einem Barcode oder QR-Code. Hierzu kann bspw. eine Farb- oder Codeerkennung mittels eines Code-Scanners und/oder einer Kamera oder ähnliches bereitgestellt werden. Wichtig bei der Farberkennung ist es, die Beleuchtung des Erkennungsmerkmals möglichst optimal einzustellen und ggf. vor Tageslichteinflüssen zu schützen, um eine bessere Ablesung zu ermöglichen. Hierfür kann eine Verschattungseinrichtung vorteilhaft kombiniert werden. Aufgrund der Orientierungsfreiheit erlaubt insbesondere die Verwendung eines QR-Codes eine hohe Erkennungssicherheit.

Der Fachmann wird die Sensoreinrichtung so ausgestalten können, dass eine optimale Positionierung des Sensors mit Bezug auf das Erkennungsmerkmal gegeben ist, und so zum Beispiel eine Distanz zwischen Sensor und Erkennungsmerkmal gewählt wird, die eine gute Merkmalserkennung erlaubt.

In einer besonders bevorzugten Ausführungsform ist die Spritzenkolbenplatte einer Spritze zur Verwendung in Spritzenpumpen mit einem QR Code als Erkennungsmerkmal bedruckt. Alternativ kann ein QR Code auch als eine Aufklippund/oder Aufsteck-Lösung auf der Spritzenkolbenplatte bereitgestellt werden.

Die Auswahl der hierin genannten Sensoren hängt von den Anforderungen der spezifischen Anwendung und den gewählten Erkennungsmerkmalen ab, und verschiedene Technologien können je nach Kontext bevorzugt werden. Ebenso können die genannten Sensoren in der Sensoranordnung kombiniert werden, um mehrere Messgrößen gleichzeitig zu erfassen oder eine redundante Erfassung sicherzustellen.

In einer weiteren Ausführungsform der medizinische Vorrichtung ist die Steuereinrichtung eingerichtet, die Antriebseinheit anweisen zu können, die Verabreichung des Medikaments zu stoppen oder zu modifizieren, insbesondere falls eine Unstimmigkeit oder ein Fehler bei der Identifikation des zuordenbaren, klassifizierbaren und/oder personalisierbaren Einmalartikels erkannt wird.

In einer weiteren Ausführungsform der medizinischen Vorrichtung ist die Steuereinrichtung eingerichtet, eine Alarmfunktion zu aktivieren, wenn das erfasste maschinenlesbares Erkennungsmerkmal des Einmalartikels nicht mit vordefinierten Informationen übereinstimmt oder wenn ein Fehler bei der Identifikation auftritt.

Nach einem dritten Aspekt der vorliegenden Erfindung, betrifft die Erfindung ein Maschinenlesbares Erkennungsmerkmal für Einmalartikel in einer medizinischen Vorrichtung, insbesondere Spritzenpumpe. Ein erfindungsgemäßes Maschinenlesbares Erkennungsmerkmal ist so bereitgestellt, dass es geeignet ist zur formgebundenen Kodierung, insbesondere einer 3D Struktur, zur Kodierung mittels einer elektronisch-physikalischen Messgröße, insbesondere einer Leitfähigkeit, einer Magnetisierung und/oder einer Drahtloswelle, insbesondere einem RFID-Tag, und/oder zur Kodierung mittels eines optischen Codes, insbesondere einer Farbe und/oder einem Muster, einem Barcode oder QR-Code.

Ein Maschinenlesbares Erkennungsmerkmal für Einmalartikel nach der vorliegenden Erfindung gemäß dem dritten Aspekt ist bevorzugt ein Maschinenlesbares Erkennungsmerkmal für einen Einmalartikel gemäß der vorliegenden Erfindung und gemäß dem ersten Aspekt. Ein Maschinenlesbares Erkennungsmerkmal für Einmalartikel nach der vorliegenden Erfindung gemäß dem dritten Aspekt ist bevorzugt ein Maschinenlesbares Erkennungsmerkmal für einen Einmalartikel zur Verwendung in einer medizinischen Vorrichtung gemäß der vorliegenden Erfindung und gemäß dem zweiten Aspekt.

Ein Erkennungsmerkmal nach der vorliegenden Erfindung ist bevorzugt so geformt, dass es an einem vorbestimmten Teilabschnitt eines Einmalartikels lösbar oder unlösbar angebracht werden kann. Dies erlaubt das Vorkonfigurieren eines Einmalartikels mittels des Erkennungsmerkmals, zum Beispiel einer Spritze zur Verwendung in Spritzenpumpen mit Hilfe eines Aufsteckbaren oder Aufklappbaren Miniatur Shields oder anderen Erkennungsmerkmalen bis unmittelbar vor der Verwendung. Insbesondere können mit einem erfindungsgemäßen Erkennungsmerkmal auch Einmalartikel individualisiert werden, die als Massenartikel verfügbar sind und werksseitig nicht speziell für eine Verwendung in einem erfindungsgemäßen System bestimmt sind.

Nach einem vierten Aspekt der vorliegenden Erfindung, betrifft die Erfindung ein System zur Erkennungsmerkmal abhängigen, insbesondere personalisierten, Medikamentenverabreichung, insbesondere für medizinische Vorrichtungen wie Spritzenpumpen, umfassend: eine Speichereinheit, die bevorzugt Informationen zu Komponenten des Systems und/oder eine Medikamentendatenbank, in welcher Daten zu Medikamenten hinterlegt sind und/oder eine Patientendatenbank, in welcher Daten zum Patienten hinterlegt sind umfasst.

Die Speichereinheit kann lokal in der Vorrichtung und/oder außerhalb der Vorrichtung vorgesehen sein, wobei eine außerhalb der Vorrichtung vorgesehen Speichereinheit insbesondere eine Netz-, Server- und/oder Cloud-basierte Speichereinheit umfasst.

Das System umfasst weitere eine medizinische Vorrichtung, bevorzugt nach der vorliegenden Erfindung, die eine Kommunikationsschnittstelle zur Übermittlung der erfassten Informationen aufweist. Die Kommunikationsschnittstelle ist dabei bevorzugt vorgesehen und eingerichtet, die erfassten Informationen an eine Speichereinheit zu übermitteln und/oder Daten aus der Speichereinheit abzurufen. Insbesondere kann eine solche Kommunikationsschnittstelle eingerichtet sein, mit einer außerhalb der Vorrichtung vorgesehenen Speichereinheit insbesondere eine Netz-, Server- und/oder Cloud-basierten Speichereinheit, zu kommunizieren. Eine derartige Kommunikationsschnittstelle kann dabei Kommunikations- und Serverprotokolle umfassen und/oder auch eine LAN, WLAN oder andere kabelgebundenen und/oder kabelungebundenen Schnittstellen aufweisen.

Das System umfasst noch weiter einen medizinischer Einmalartikel für eine medizinische Vorrichtung insbesondere Spritzenpumpe, wobei der Einmalartikel insbesondere eine Spritze zur Verwendung in Spritzenpumpen ist, und ein maschinenlesbares Erkennungsmerkmal umfasst, das ein Merkmal des Einmalartikels ist oder ein an einem vorbestimmten Teilabschnitt des Einmalartikels lösbar oder unlösbar, insbesondere werkseitig, angebracht ist.

In einer besonders bevorzugten Ausführungsform des Systems nach der vorliegenden Erfindung, ist die Steuereinheit dafür vorgesehen und eingerichtet, anhand des detektierten und damit identifizierten Einmalartikels eine Medikamentenauswahlmöglichkeit in der Medikamentendatenbank zu filtern und/oder zu begrenzen; und/oder eine Auswahlmöglichkeit eines weiteren Einmalartikels zu filtern und/oder zu begrenzen; und/oder bei einer Einstellung einer Therapie der medizinischen Vorrichtung mit vorbestimmter Kombination von Medikament oder Medikamentenkonzentration zu Einmalartikel, einen Alarm auszugeben und/oder einen Start der Therapie zu unterbinden.

In einer besonders bevorzugten Ausführungsform des Systems nach der vorliegenden Erfindung, ist die Steuereinheit dafür vorgesehen und eingerichtet, anhand des detektierten und damit identifizierten Einmalartikels Einstellung eines Funktionsmodus der Pumpe, bspw. die Abgabegeschwindigkeit eines Medikaments und/oder die Vorschubgeschwindigkeit eines Spritzenkolbens; Beschränkung von Funktionen, Beschränkung der Medikamentendatenbank oder ähnliches vorzunehmen.

Die Speichereinheit umfasst bevorzugt Informationen zu Komponenten des Systems, bspw. Informationen zur medizinischen Vorrichtung, z.B. zur Pumpe, Pumpenleistung, Pumpen-Spezifikation oder weitere Vorrichtungsmerkmale.

Gleichzeitig oder alternativ umfasst die Speichereinheit bevorzugt eine Patientendatenbank, in welcher Daten zum Patienten hinterlegt sind, bspw. eine Patienten-ID, Behandlungsschemata und Medikationsdetails, oder ähnliches. Es können auch weitere Informationen in der Patientendatenbank hinterlegt sein, insbesondere persönliche und/oder gesundheitsbezogene Patientendaten, wie Alter, Gewicht, Name, usw.

Gleichzeitig oder alternativ umfasst die Speichereinheit bevorzugt eine Medikamentendatenbank, in welcher Daten zu Medikamenten hinterlegt sind, bspw. Dosierungsschemata, Ablaufdatum, oder ähnliches.

Die Kommunikationsschnittstelle übermittelt in dem erfindungsgemäßen System erfasste Informationen bevorzugt an eine Speichereinheit und/oder ruft Daten aus der Speichereinheit ab. Dabei erfolgt der Abruf von Daten bevorzugt abhängig von dem anhand des maschinenlesbaren Erkennungsmerkmals erkannten medizinischer Einmalartikels für eine medizinische Vorrichtung, die von der Kommunikationsschnittstelle an die Speichereinheit übermittelt werden.

Dabei ist der Einmalartikel und/oder die medizinische Vorrichtung und/oder das maschinenlesbare Erkennungsmerkmal, bevorzugt ein Einmalartikel und/oder eine medizinische Vorrichtung und/oder ein maschinenlesbares Erkennungsmerkmal nach der vorliegenden Erfindung.

In einem erfindungsgemäßen System erfolgt die Erkennung und Zuordnung von zuordenbaren, klassifizierbaren und/oder personalisierbaren Einmalartikeln durch ein spezifisches maschinenlesbares Erkennungsmerkmal, welches durch die Erkennungseinrichtung der medizinischen Vorrichtung erkannt wird. Anhand dieser Erkennung und Zuordnung und des Einmalartikel können insbesondere vorbestimmte Informationen dem Einmalartikel zugewiesen werden, die basierend auf der Erkennung des Erkennungsmerkmals aus der Speichereinheit abgerufen wurden. Die Speichereinheit, umfasst dabei bevorzugt Informationen zu Komponenten des Systems und/oder eine Medikamentendatenbank, in welcher Daten zu Medikamenten hinterlegt sind und/oder eine Patientendatenbank, in welcher Daten zum Patienten hinterlegt sind.

Die Erkennung des Erkennungsmerkmals durch die medizinische Vorrichtung erlaubt eine eindeutige Abfrage von vorbestimmten Informationen aus der Cloud-basierten Anwendungsdatenbank anhand des Erkennungsmerkmals. Mit derartigen Informationen und basierend auf der eindeutigen Zuweisung des Einmalartikels können, bspw. eine Patienten-ID, Behandlungsschemata und Medikamentendetails, aus der Cloud-basierten Anwendungsdatenbank abgerufen werden, die für die sichere Behandlung des Patienten notwendig und/oder hilfreich sein können.

Gleichzeitig können Informationen zu Komponenten des Systems, zur Pumpe, Sicherheitsmerkmale wie Uhrzeit, Datum, Seriennummer und Pumpe verwendet werden, um eine falsche Therapie am Patienten zu verhindern.

Neben dem Abrufen von Informationen aus der Cloud-basierten Anwendungsdatenbank, kann eine Vorrichtung und/oder ein System nach der vorliegenden Erfindung ebenfalls dazu eingerichtet und/oder vorgesehen sein, Informationen, bspw. Therapie-, Medikamenten-, System-, Vorrichtungs- oder Patienteninformationen in die Cloud-basierten Anwendungsdatenbank zu schreiben. Beispielsweise kann nach erfolgter Erkennung des eingelegten Einmalartikels, dieser Einmalartikel einem bestimmten Patienten und/oder einer bestimmten Patienten-ID in der Cloud-basierten Anwendungsdatenbank zugeordnet werden. Alternativ oder zusätzlich können Informationen über den Einmalartikel, zum Beispiel ein Verwendungsdatum, Ablaufdatum, ein Restvolumen, oder ähnliches, in der Cloud-basierten Anwendungsdatenbank dem identifizierten Einmalartikel zugeordnet werden.

Der lokale Datenabgleich zwischen medizinischer Vorrichtung und Cloud-basierten Anwendungsdatenbank gewährleistet dabei vorteilhafterweise die Konsistenz der Informationen und die Zuordnung des anhand des Erkennungsmerkmals eindeutig identifizierten Einwegartikels. Zusammen mit den Informationen über die medizinische Vorrichtung, können so alle Systemkomponenten einander - und ggf. auch dem Patienten - eindeutig zugeordnet werden.

Ausgehend von einem solchen erfindungsgemäßen System sind verschiedene erfindungsgemäße Fortbildungen möglich, die von der vorliegenden Erfindung umfasst sind. Insbesondere kann eine Cloud-basierte Datenbank mit optionalem Abruf an der Vorrichtung.

Eine Cloud-Datenbank kann insbesondere Anwendungen, Patienteninformationen, Behandlungsschemata und Medikamentendetails als vordefinierte Daten umfassen und diese mittels Netzwerkverbindung, ggf. drahtlos, an die medizinische Vorrichtung übertragen. Insbesondere kann eine solche Übertragung durch die Erkennung des Erkennungsmerkmals eines Einmalartikels ausgelöst oder ermöglicht sein. Sodann kann der zuordenbare, klassifizierbare und/oder personalisierbare Einmalartikel ebenfalls vordefinierte Informationen umfassen, bspw. eine Patienten-ID, ein Medikament und Details über Therapie und Behandlungsschema. Diese Daten können mittels Erkennung des Einmalartikels anhand des Erkennungsmerkmal der medizinischen Vorrichtung zugeordnet werden und an diese übertragen oder von dieser mittels der Erkennungseinrichtung ausgelesen werden. Sodann kann ein Datenabgleich von Informationen aus der Cloud-Datenbank und von Informationen des zuordenbaren, klassifizierbaren und/oder personalisierbaren Einmalartikels, sowie ggf. auch weitere manuell eingebbare Informationen, abgeglichen werden. Ein solcher Abgleich kann lokal auf der medizinischen Vorrichtung und/oder in einer entsprechenden Server- oder Cloud-Anwendung erfolgen. Insbesondere kann die medizinische Vorrichtung lokale Daten mit der Cloud abgleichen und den zuordenbaren, klassifizierbaren und/oder personalisierbaren Einmalartikel den Patienteninformationen zuordnen.

In einer anderen Ausführungsform kann eine Cloud-basierte Datenbank mit Schlüsselcode-Übermittlung bereitgestellt sein. So kann ein Schlüsselcode in einer Cloud-Anwendung generiert und an die medizinische Vorrichtung übermittelt werden. Der Schlüsselcode kann anhand verschiedener vordefinierter Informationen erstellt werden, insbesondere Informationen über die medizinische Vorrichtung, zum Beispiel eine Seriennummer, oder Informationen über den Patienten, insbesondere eine eindeutige Patienten-ID, Informationen über Medikament und/oder Behandlungsschema. Der Schlüsselcode kann dann an die medizinische Vorrichtung ausgegeben werden. Die medizinische Vorrichtung kann dabei so eingerichtet sein, dass nur ein Einmalartikel akzeptiert wird, der im Abgleich zu dem Schlüsselcode übereinstimmt. Dabei kann ein zuordenbarer, klassifizierbarer und/oder personalisierbarer Einmalartikel ebenfalls vordefinierte Informationen enthalten, bspw. auf einem Erkennungsmerkmal, oder nach Identifizierung des Einmalartikels solchen Informationen zugeordnet sein. So kann der zuordenbare, klassifizierbare und/oder personalisierbare Einmalartikel lokal oder in der Cloud-Anwendung verschiedenen vordefinierten Daten zugeordnet werden. Das maschinenlesbare Erkennungsmerkmal des Einmalartikels wird dann mit dem Schlüsselcode abgeglichen, um die Akzeptanz durch die medizinische Vorrichtung sicherzustellen.

Weiterhin kann ebenfalls eine Cloud-basierte Datenbank mit Over-the-Air-Datenabgleich bereitgestellt werden. Dabei enthält die Cloud-Datenbank wiederum vordefinierte Informationen, einschließlich Informationen der medizinischen Vorrichtung, Patienteninformationen und eine Patienten-ID und Behandlungsdetails. Nach einlegen des Einmalartikels in die medizinische Vorrichtung wird dieser anhand des Erkennungsmerkmals erkannt und dem Gerät zugeordnet. Zum einen kann die medizinische Vorrichtung einen Abgleich der Daten der Cloud-Datenbank vornehmen, zum anderen kann ein durch die Cloud erzeugter Schlüsselcode abrufbar sein, der dann zur Bestätigung händisch in der medizinischen Vorrichtung eingegeben werden kann. Dabei kann der Datenabgleich zwischen Cloud und der medizinischen Vorrichtung bevorzugt drahtlos (Over the Air) erfolgen. Durch Eingabe eines Schlüsselcodes manuell oder über die Cloud an die medizinische Vorrichtung, kann vorteilhaft das maschinenlesbare Erkennungsmerkmal des Einmalartikels verifiziert werden.

In einer Ausführungsform ist das erfindungsgemäße System bevorzugt derart eingerichtet, dass die Erkennungseinrichtung zugeordnete, klassifizierte und/oder personalisierte Merkmale des Einmalartikels erfasst und sicherstellt, dass nur Einmalartikel akzeptiert werden, deren Merkmale mit den Daten in der Cloud übereinstimmen.

In einer Ausführungsform ist das erfindungsgemäße System bevorzugt derart eingerichtet, dass Erkennungseinrichtung sicherstellt, dass der lokale Datenabgleich nur bei erfolgreicher Überprüfung des personalisierten Einmalartikels mit dem zugehörigen Schlüsselcode durchgeführt wird.

In einer Ausführungsform ist das erfindungsgemäße System derart eingerichtet, dass die Cloud-basierte Anwendungsdatenbank weitere Informationen wie Anwendungszeitpunkt und Intervall enthält und diese Informationen in den lokalen Datenabgleich einbezogen werden, um eine präzise und personalisierte Medikamentenverabreichung zu gewährleisten.

Nach einem fünften Aspekt der vorliegenden Erfindung, betrifft die Erfindung ein Verfahren zur personalisierten Medikamentenverabreichung in einem medizinischen System, insbesondere für Spritzenpumpen. Ein erfindungsgemäßes Verfahren umfasst wenigstens die folgenden Schritte:
- ein Bereitstellen einer medizinischen Vorrichtung, bevorzugt einer Vorrichtung nach einem der vorstehenden Ansprüche,
- ein Einbringen eines Einmalartikels für die medizinische Vorrichtung, bevorzugt ein Einmalartikel nach einem der vorstehenden Ansprüche, umfassend ein Maschinenlesbares Erkennungsmerkmal, wobei das maschinenlesbare Erkennungsmerkmal ein Merkmal des Einmalartikels ist oder ein an einem vorbestimmten Teilabschnitt des Einmalartikels lösbar oder unlösbar, insbesondere werkseitig, angebracht ist;

- ein Erkennen des Einmalartikels, bevorzugt mittels einer Erkennungseinrichtung der medizinischen Vorrichtung, mittels des maschinenlesbaren Erkennungsmerkmals des Einmalartikels;
- ein Identifizieren des Erkennungsmerkmals des Einmalartikels auf Basis der erfassten Informationen;
- ein Steuern einer Antriebseinheit, bevorzugt mittels einer Steuereinrichtung der medizinischen Vorrichtung, zur automatisierten Verabreichung eines Medikaments gemäß erkannter Informationen des Einmalartikels.

Es sei darauf hingewiesen, dass die oben angegebenen Schritte nicht zwangsläufig in der gegebenen Reihenfolge durchgeführt werden müssen. Die bereitgestellten Schritte können in jeder anderen geeigneten Reihenfolge durchgeführt werden.

Die oben angegebene Reihenfolge kann jedoch für bestimmte Ausführungsformen des Verfahrens gelten.

Es wird unmittelbar von einer fachkundigen Person erkannt, dass eine Eigenschaft, Ausführungsform, Wirkung oder Vorteil, die hier in Verbindung mit dem erfinderischen medizinischen Einmalartikel für eine medizinische Vorrichtung, insbesondere eine Spritze zur Verwendung in einer Spritzenpumpe, der medizinischen Vorrichtung, insbesondere einen Spritzenpumpe, zur automatisierten Verabreichung von Medikamenten an einen Patienten, dem maschinenlesbaren Erkennungsmerkmal für Einmalartikel in einer medizinischen Vorrichtung, und dem System zur Erkennungsmerkmal abhängigen, insbesondere personalisierten, Medikamentenverabreichung, beschrieben wird, auch eine Eigenschaft, Ausführungsform, Wirkung oder Vorteil des erfinderischen Verfahrens sein kann, und umgekehrt.

In einer Ausführungsform kann das Verfahren nach der vorliegenden Erfindung weiter umfassen ein Abgleichen von vordefinierten Informationen, die anhand eines Erkennungsmerkmals des Einmalartikels identifiziert wurden mit vordefinierten Informationen in einer Cloud-basierte Anwendungsdatenbank.

In einer Ausführungsform kann das Verfahren nach der vorliegenden Erfindung weiter umfassen ein Sicherstellen mittels einer Erkennungseinrichtung, dass der lokale Datenabgleich nur bei erfolgreicher Überprüfung des personalisierten Einmalartikels mit dem zugehörigen Schlüsselcode durchgeführt wird.

In einer Ausführungsform kann das Verfahren nach der vorliegenden Erfindung weiter umfassen, dass die Cloud-basierte Anwendungsdatenbank zusätzliche Informationen wie Anwendungszeitpunkt und Intervall enthält und diese Informationen in den lokalen Datenabgleich einbezogen werden, um eine präzise und personalisierte Medikamentenverabreichung zu gewährleisten.

Alle beschriebenen Ausführungsformen der vorliegenden Erfindung haben den Vorteil, dass sowohl kurzfristig als auch mittel- bis langfristig signifikante Verbesserungen gegenüber den im Stand der Technik bekannten Lösungen bestehen. Das Erkennungsmerkmal erhöht besonders vorteilhaft die Sicherheit bei der Auswahl und Anwendung von Medizinprodukten durch die Möglichkeit der Vorparametrisierung. Zudem geht mit der vorliegenden Erfindung nur ein relativ geringer Kostenmehraufwand für den Einmalartikel einher. Die vorliegende Erfindung ermöglicht ebenfalls eine Integration in verschiedene Anwendungsbereiche wie Homecare, da die Vermeidung einer Fehlzuordnung von Medikament zu Patienten vermieden wird, und somit ungeschultes Personal leichter die vorliegende Erfindung anwenden kann. Weiterhin können vordefinierte Informationen leichter übertragen werden, bspw., die Übertragung von Therapiemustern auf verschiedene medizinische Vorrichtungen erlauben, die medizinische Vorrichtung leicht zu tauschen. Die mögliche Cloud-Anbindung gewährleistet zudem eine sichere und effiziente Datenübertragung, unabhängig von der Standortentfernung. Insgesamt tragen die Sicherheitsmerkmale dazu bei, eine korrekte Identifikation von personalisierten Einmalartikeln sicherzustellen und erhöhen somit die Patientensicherheit.

### Beschreibung der Figuren

Die vorliegende Erfindung wird im Folgenden unter Bezugnahme auf die Zeichnungen näher erläutert, aus denen weitere Merkmale, Ausführungsformen und Vorteile entnommen werden können, und in denen:
FIG. 1A, 1B, 1C, und 1D zeigen schematische Dartstellungen von Ausführungsformen von erfindungsgemäßen Einmalartikeln und Erkennungsmerkmalen;
FIG. 2 zeigt eine schematische Darstellung einer Ausführungsform einer medizinischen Vorrichtung gemäß der vorliegenden Erfindung.

Die in der Beschreibung, den Ansprüchen, Beispielen und/oder den Figuren offengelegten Merkmale der vorliegenden Erfindung können sowohl einzeln als auch in beliebiger Kombination dazu dienen, die Erfindung in verschiedenen Formen zu realisieren.

In den in den Figuren gezeigten Ausführungsformen werden Elemente, die in Funktion ähnlich oder identisch sind, mit gleichen Bezugszeichen bezeichnet. Es sei darauf hingewiesen, dass die Figuren möglicherweise nicht maßstabsgetreu zueinander sind.

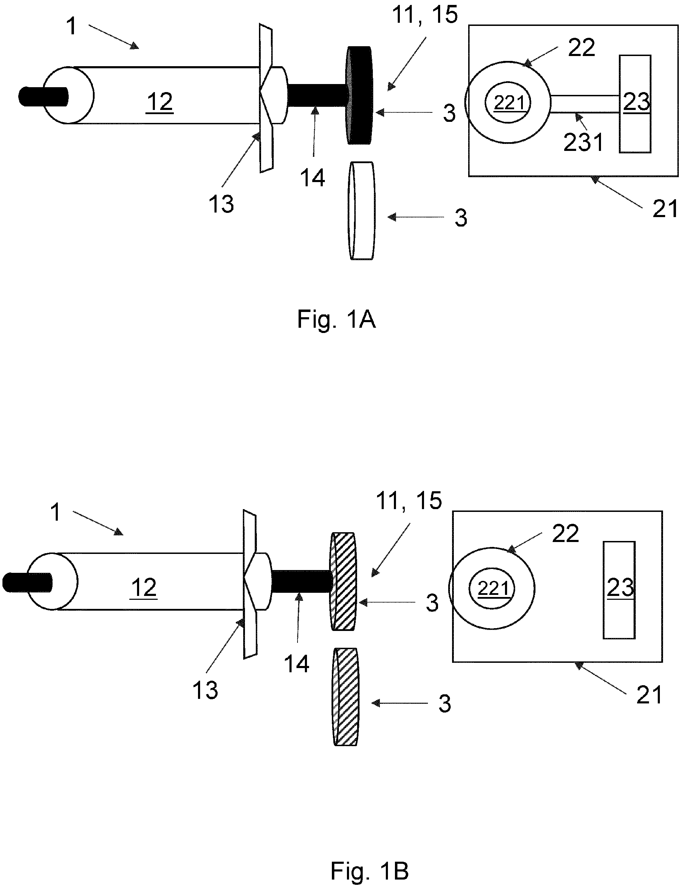

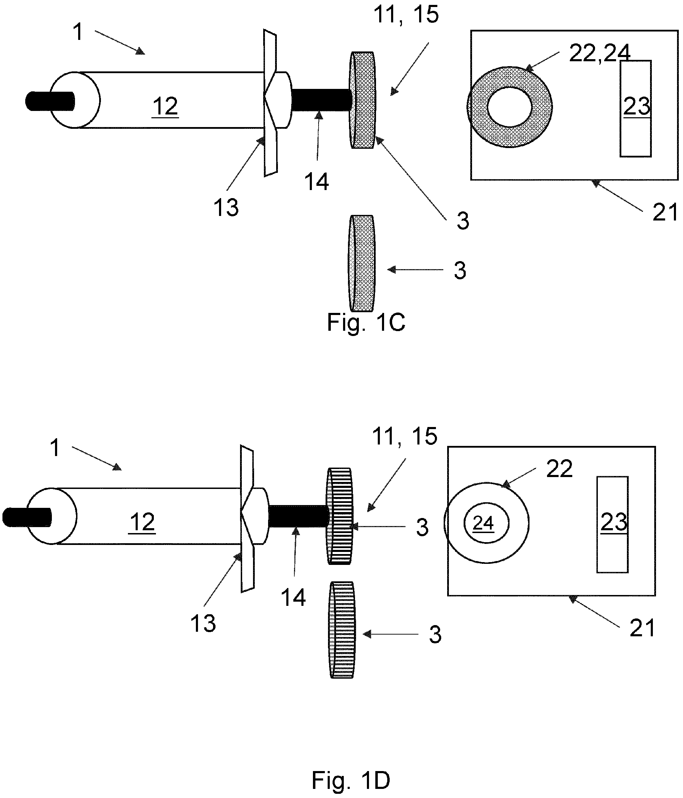

FIG. 1 zeigt eine schematische Darstellung einer ersten Ausführungsform eines medizinischen Einmalartikels 1 für eine medizinische Vorrichtung 2, in Form einer Spritzenpumpe 2. Der Einmalartikel 1 ist als Spritze zur Verwendung in Spritzenpumpen 2 dargestellt und umfasst ein maschinenlesbares Erkennungsmerkmal 3. Die Spritze zur Verwendung in Spritzenpumpen 1 umfasst einen Spritzenzylinder 12 in dem das zu verabreichende Medikament beinhaltet ist. Zum manuellen Gebrauch sind üblicherweise an dem Zylinder 12 Spitzenzylinderflügel 13 angeformt, um ein manuelles Verabreichen eines Medikaments mit dem Spritzenpumpe 2, bspw. mit aufgesetzter Kanüle zu erleichtern. Diese Spitzenzylinderflügel 13 dienen zusätzlich auch dazu, den Spritzenkolben 14 in der Spritzenpumpe 2 zu halten und einzuklemmen.

In den Spritzenzylinder 12 ist ein Stößel 14, in der Form eines Spritzenkolbens 14 eingeführt, an dessen Ende eine Spritzenkolbenplatte 15 angeformt ist. Wird bestimmungsgemäß die Spritzenkolbenplatte 15 gedrückt, wird hierdurch der Spritzenkolben 14 in dem Zylinder 12 bewegt und das Medikament aus der Spritze 1 zur Verwendung in Spritzenpumpen 2 gedrückt. In einer medizinischen Vorrichtung wie einem Spritzenpumpe 2, ist üblicherweise eine Antriebseinheit 21, insbesondere ein Antriebskopf 21, vorgesehen, der zur Beaufschlagung der Spritzenkolbenplatte 15 der eingesetzten Spritze zur Verwendung in Spritzenpumpen 2 mit Druck, auf die Spritzenkolbenplatte 15 gedrückt wird. In der gezeigten Ausführungsform ist als das Erkennungsmerkmal 3 eine über die Spritzenkolbenplatte 15 überziehbare Gummitülle 3 vorgesehen, die farbig ausgestaltet ist. Dieses maschinenlesbare Erkennungsmerkmal 3 kann also einem vorbestimmten Teilabschnitt 11, hier nämlich die Spritzenkolbenplatte 15, des Einmalartikels 1 angebracht werden. Alternativ, könnte auch der gesamte Einmalartikel 1, oder ein Teil davon, bspw. die Spritzenkolbenplatte 15 selbst, ein solches ein maschinenlesbares Erkennungsmerkmal 3 aufweisen, beispielsweise in Form eines farbigen Einmalartikels 1 oder einer farbigen Spritzenkolbenplatte 15. Damit wäre ein unlösbar mit der Spritzenkolbenplatte 15 verbundenes Merkmal 3 verwirklicht. Dies kann insbesondere werksseitig bereits vorgesehen sein. In der gezeigten Ausführungsform ist der vorbestimmte Teilabschnitt 11, also die Spritzenkolbenplatte 15 dem Antriebskopf 21, und dem Membranteller 22 der Spritzenpumpe 2 zugewandt.

In FIG. 1A ist schematisch ebenfalls der Antriebskopf 21 einer Spritzenpumpe 2 dargestellt. Erkennbar ist eine Erkennungseinrichtung 23, die zur Identifikation der personalisierten Einmalspritze zur Verwendung in Spritzenpumpen 2 vorgesehen ist. Die Erkennungseinrichtung 23 umfasst eine nicht dargestellte Sensoranordnung 24, die dazu eingerichtet ist das maschinenlesbare Erkennungsmerkmal 3 des Einmalartikels 1 zu erfassen.

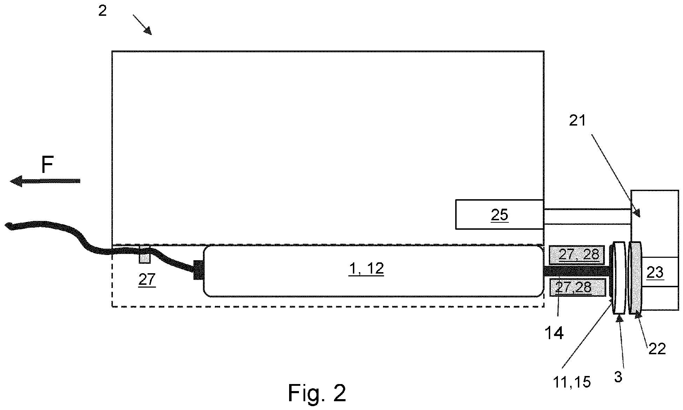

FIG. 2 zeigt eine schematische Darstellung einer Ausführungsform eines Spritzenpumpes 2 nach der vorliegenden Erfindung auf den im Folgenden ebenfalls

Bezug genommen wird. Dort ist die Spritze 1 in den Spritzenpumpe 2 eingesetzt. Der Spritzenpumpe 2 umfasst eine Steuereinrichtung 25, die mit der Sensoranordnung verbunden ist und auf Basis der erfassten Informationen das maschinenlesbare Erkennungsmerkmal 3 des Einmalartikels 1 identifiziert. Der Spritzenpumpe 2 umfasst weiter eine Antriebseinheit 21, die durch die Steuereinrichtung 25 gesteuert wird, um das Medikament gemäß den erkannten Informationen des Einmalartikels 1 automatisiert an den Patienten zu verabreichen. Die Antriebseinheit 21, insbesondere der Antriebskopf 21 oder Membranteller 22, umfasst die Erkennungseinrichtung 23 als Teil der Antriebseinheit 21, insbesondere des Antriebskopfs 21.

Der Spritzenpumpe 2 umfasst auch einen Haltemechanismus 27, insbesondere ein Klemm-Mechanismus der die Spritze zur Verwendung in Spritzenpumpen 2im Spritzenpumpe 2 hält. Insbesondere werden die Spritzenzylinderflügel 13 am Spritzenzylinder 12 eingeklemmt. Dieser Haltemechanismus kann 27 die Erkennungseinrichtung 23 umfassen, dient jedoch in der in FIG. 2 gezeigten Ausführungsform als Verschattungseinrichtung 28 zur wenigstens partiellen Verschattung des Erkennungsmerkmals 3, das in FIG. 2 aufgesetzt auf die Spritzenkolbenplatte 15 dargestellt ist. Hier ist also die Verschattungseinrichtung 28 als Teil des Haltemechanismus 27 zur Halterung der Spritze 1 im Spritzenpumpe 2 ausgebildet. Alternativ kann bspw. auch ein Klemmmechanismus für die Spritzenzylinderflügel 13 am Spritzenzylinder 12 auch als Verschattungseinrichtung eingerichtet sein.

In FIG. 1A ist weist der Membranteller 22, eine Sensorsignalöffnung 221 auf. Diese kann insbesondere durch ein sensorsignaldurchgängiges, insbesondere durchsichtiges Material, durch ein integriertes Fenster, eine optische Linse oder eine Materialauslassung und/oder Materialaussparung gebildet sein. Hier ist die Erkennungseinrichtung 23 mit der Sensoranordnung 24 verbunden und weist einen flexibel geformten, optischen Leiter, hier einen Lichtleiter 231 auf, der das erkannte Signal zu einer Erkennungseinrichtung 23 leitet. Diese Erkennungseinrichtung 23 weist ebenfalls eine Lichtquelle auf, die durch den Lichtleiter 231 und die Öffnung 221 das Licht gezielt dem Erkennungsmerkmal 3 zuführt. Damit ist das Erkennungsmerkmal - insbesondere auch durch die Verschattung 28 - optimal beleuchtet und die Sensoranordnung 24, bspw. umfassend in diesem Fall eine Farbdetektions-Kamera, kann das Erkennungsmerkmal optimal auslesen. Dabei ist die Erkennungseinrichtung 23 so angeordnet, dass sie zur zentrischen Erfassung des Erkennungsmerkmals 3 auf der Spritzenkolbenplatte 15 geeignet ist.

Alternativ zum flexiblen Lichtleiter 231 könnte auch eine Optik vorgesehen sein, die eine kabelgebundene LED aufweist. Dabei kann die LED direkt am Membranteller 22 angebracht sein.

In FIG. 1B ist eine weitere Ausführungsform der Erfindung dargestellt. Hierbei ist ein das Erkennungsmerkmal 3 als eine formgebundene Kodierung, insbesondere als geriffelte 3D Struktur entweder auf der Spritzenkolbenplatte 15 oder als Gummitülle vorgesehen. Demgemäß umfasst die Erkennungseinrichtung 23 eine andere Sensorik 24, bspw., in Form eines resistiven Touchsensors 24 oder eines mechanischen Abgriffsensors 24. Der Membranteller 22 kann in der Öffnung 221 bereits eine solche Sensorik 24 aufweisen, die sodann die Signale an eine entsprechende elektronische Erkennungseinrichtung 23 weiterleiten kann.

In FIG. 1C ist eine weitere Ausführungsform der Erfindung dargestellt. Hierbei ist das Erkennungsmerkmal 3 als eine die Spritzenkolbenplatte 15 umlaufende RFID-Antenneneinheit ausgebildet. Diese kann wiederum fest montiert, oder als überziehbare Gummitülle 3 oder nach/an-montierbare Kolbenplatte 15 ausgestaltet sein. Demgemäß umfasst die Erkennungseinrichtung 23 eine andere Sensorik 24, bspw., in Form einer RFID-Antenneneinheit 24. Der hier massive ausgestaltete Membranteller 22 kann eine solche RFID-Antenneneinheit 24, bspw. als umlaufende Ausgestaltung aufweisen, die sodann die Signale an eine entsprechende elektronische Erkennungseinrichtung 23 weiterleiten kann.

In FIG. 1D ist eine weitere Ausführungsform der Erfindung dargestellt. Hierbei ist das Erkennungsmerkmal 3 als ein QR-Code ausgestaltet, der entweder in die Spritzenkolbenplatte 15 eingebracht ist, oder auf die überziehbare Gummitülle 3 aufgedruckt ausgestaltet ist. Demgemäß umfasst die Erkennungseinrichtung 23 eine andere Sensorik 24, bspw., in Form eines QR-Code Readers 24. Der Membranteller 22 kann eine solche QR-Reader-Einheit 24, bspw. in einer Aussparung 221 aufweisen, die sodann die Signale an eine entsprechende elektronische Erkennungseinrichtung 23 weiterleiten kann.

Wie in Figuren 1A, 1B, 1C und 1D gezeigt kann somit die medizinische Vorrichtung 2 nach der vorliegenden Erfindung bereitgestellt werden, wobei die Sensoranordnung 24 geeignet ist, das maschinenlesbare Erkennungsmerkmal 3 zu detektieren und insbesondere ausgewählt ist aus einer Sensoranordnung 24 zur Detektion
einer formgebundenen Kodierung, insbesondere einer 3D Struktur,
- einer elektronisch-physikalischen Messgröße, insbesondere einer Leitfähigkeit, einer Magnetisierung und/oder einer Drahtloswelle, insbesondere einem RFID-Tag, und/oder
- einem optischen Code, insbesondere einer Farbe und/oder einem Muster, einem Barcode oder QR-Code.

Die in den Figuren dargestellten Ausführungsformen können sich auf bevorzugte Ausführungsformen beziehen, während alle Elemente und Merkmale, die in Verbindung mit den Ausführungsformen beschrieben sind, soweit angebracht, in Kombination mit jeder anderen Ausführungsform und jedem anderen Merkmal verwendet werden können, wie hierin besprochen, insbesondere in Bezug auf jede andere zuvor besprochene Ausführungsform.

### Bezugszeichenliste

- 1: medizinischer Einmalartikel
- 11: Teilabschnitt
- 12: Spritzenzylinder
- 13: Spritzenzylinderflügel
- 14: Stößel/Spritzenkolben
- 15: Spritzenkolbenplatte
- 2: medizinische Vorrichtung
- 21: Antriebseinheit, Antriebskopf
- 22: Membranteller
- 221: Sensorsignalöffnung
- 23: Erkennungseinrichtung
- 231: optischer Leiter
- 24: Sensoranordnung
- 25: Steuereinrichtung
- 26: Kommunikationsschnittstelle
- 27: Haltemechanismus
- 28: Verschattungseinrichtung
- 3: Erkennungsmerkmal
- 4: System

## Patentansprüche

1. Medizinischer Einmalartikel (1) für eine medizinische Vorrichtung (2), insbesondere Spritzenpumpen, wobei der Einmalartikel (1) bevorzugt eine Spritze zur Verwendung in Spritzenpumpen ist, umfassend ein maschinenlesbares Erkennungsmerkmal (3), wobei das maschinenlesbare Erkennungsmerkmal (3) ein Merkmal des Einmalartikels (1) ist oder ein an einem vorbestimmten Teilabschnitt (11) des Einmalartikels (1) lösbar oder unlösbar, insbesondere werkseitig, angebracht ist.

2. Einmalartikel (1) nach einem der vorhergehenden Ansprüche, wobei der vorbestimmte Teilabschnitt (11) ein einem Antriebskopf (21), insbesondere einem Membranteller (22), einer medizinischen Vorrichtung (2) zugewandter Teil, insbesondere eine Spritzenkolbenplatte (15) ist.

3. Einmalartikel (1) nach einem der vorhergehenden Ansprüche, wobei das maschinenlesbare Erkennungsmerkmal (3) ausgewählt ist aus
- einer formgebundenen Kodierung, insbesondere einer 3D Struktur,
- einer elektronisch-physikalischen Messgröße, insbesondere einer Leitfähigkeit, einer Magnetisierung und/oder einer Drahtloswelle, insbesondere einem RFID-Tag, und/oder
- einem optischen Code, insbesondere einer Farbe und/oder einem Muster, einem Barcode oder QR-Code.

4. Medizinische Vorrichtung (2), insbesondere Spritzenpumpe (2), zur automatisierten Verabreichung von Medikamenten an einen Patienten, umfassend:
- eine Erkennungseinrichtung (23) zur Identifikation eines zuordenbaren, klassifizierbaren und/oder personalisierbaren Einmalartikels (1), umfassend eine Sensoranordnung (24), die dazu eingerichtet ist ein maschinenlesbares Erkennungsmerkmal (3) des Einmalartikels (1) zu erfassen;
- eine Steuereinrichtung (25), die mit der Sensoranordnung (24) verbunden ist und auf Basis der erfassten Informationen das maschinenlesbare Erkennungsmerkmal (3) des Einmalartikels (1) identifiziert;
- eine Antriebseinheit (21), die durch die Steuereinrichtung (25) gesteuert wird, um das Medikament gemäß den erkannten Informationen des Einmalartikels (1) automatisiert an den Patienten zu verabreichen,
wobei der Einmalartikel (1), bevorzugt ein Einmalartikel (1) nach einem der vorhergehenden Ansprüche ist.

5. Medizinische Vorrichtung (2) nach einem der vorgehenden Ansprüche, umfassend eine Aktivierungseinrichtung zur Aktivierung und Deaktivierung der Erkennungseinrichtung (23).

6. Medizinische Vorrichtung (2) nach einem der vorhergehenden Ansprüche, wobei die Antriebseinheit (21), insbesondere ein Antriebskopf (21) oder Membranteller (22), die Erkennungseinrichtung (23) umfasst und/oder die Erkennungseinrichtung (23) als Teil der Antriebseinheit (21), insbesondere eines Antriebskopfs (21) der Antriebseinheit (21), ausgebildet ist und/oder an der Antriebseinheit (21), insbesondere einem Antriebskopf (21) der Antriebseinheit (21), angebracht ist.

7. Medizinische Vorrichtung (2) nach einem der vorhergehenden Ansprüche, wobei die Medizinische Vorrichtung (2) einen Haltemechanismus (27), insbesondere Klemm-Mechanismus, zur Halterung des Einwegartikels (1) in der medizinischen Vorrichtung (2) aufweist, und wobei der Haltemechanismus (27) die Erkennungseinrichtung (23) umfasst.

8. Medizinische Vorrichtung (2) nach einem der vorhergehenden Ansprüche, wobei die Antriebseinheit (21), insbesondere ein Antriebskopf (21) oder Membranteller (22), eine Sensorsignalöffnung (221) aufweist.

9. Medizinische Vorrichtung (2) nach einem der vorhergehenden Ansprüche, wobei die Erkennungseinrichtung (23) einen mit der Sensoranordnung (24) verbundenen, bevorzugt flexibel geformten, optischer Leiter, bevorzugt Lichtleiter, (231) umfasst.

10. Medizinische Vorrichtung (2) nach einem der vorhergehenden Ansprüche, wobei die Erkennungseinrichtung (23) zur zentrischen Erfassung des Erkennungsmerkmals (3), insbesondere eines Erkennungsmerkmals (3) auf einer Spritzenkolbenplatte (15), eingerichtet ist.

11. Medizinische Vorrichtung (2) nach einem der vorhergehenden Ansprüche, insbesondere die Erkennungseinrichtung (23), umfassend eine Verschattungseinrichtung (28) zur wenigstens partiellen Verschattung des Erkennungsmerkmals (3), wobei bevorzugt die Verschattungseinrichtung (28) als Teil eines Haltemechanismus (27), insbesondere Klemm-Mechanismus, zur Halterung des Einwegartikels (1) in der medizinischen Vorrichtung (2) ausgebildet ist.

12. Medizinische Vorrichtung (2) nach einem der vorhergehenden Ansprüche, wobei die Sensoranordnung (24) geeignet ist das maschinenlesbare Erkennungsmerkmal (3) zu detektieren und insbesondere ausgewählt ist aus einer Sensoranordnung (24) zur Detektion
- einer formgebundenen Kodierung, insbesondere einer 3D Struktur,
- einer elektronisch-physikalischen Messgröße, insbesondere einer Leitfähigkeit, einer Magnetisierung und/oder einer Drahtloswelle, insbesondere einem RFID-Tag, und/oder
- einem optischen Code, insbesondere einer Farbe und/oder einem Muster, einem Barcode oder QR-Code.

13. Ein Maschinenlesbares Erkennungsmerkmal (3) für Einmalartikel (1) in einer medizinischen Vorrichtung (2), insbesondere Spritzenpumpe, geeignet
- zur formgebundenen Kodierung, insbesondere einer 3D Struktur,
- zur Kodierung mittels einer elektronisch-physikalischen Messgröße, insbesondere einer Leitfähigkeit, einer Magnetisierung und/oder einer Drahtloswelle, insbesondere einem RFID-Tag, und/oder
- zur Kodierung mittels eines optischen Codes, insbesondere einer Farbe und/oder einem Muster, einem Barcode oder QR-Code.

14. Ein System zur Erkennungsmerkmal abhängigen, insbesondere personalisierten, Medikamentenverabreichung, insbesondere für medizinische Vorrichtungen (2) wie Spritzenpumpen, umfassend:
- eine Speichereinheit, die bevorzugt Informationen zu Komponenten des Systems (4) und/oder eine Medikamentendatenbank, in welcher Daten zu Medikamenten hinterlegt sind und/oder eine Patientendatenbank, in welcher Daten zum Patienten hinterlegt sind umfasst;
- eine medizinische Vorrichtung (2) umfassend eine Kommunikationsschnittstelle (26) zur Übermittlung der erfassten Informationen;
- einen medizinischer Einmalartikel (1) für eine medizinische Vorrichtung (1) insbesondere Spritzenpumpe, wobei der Einmalartikel (1) insbesondere eine Spritze zur Verwendung in Spritzenpumpen ist, umfassend ein maschinenlesbares Erkennungsmerkmal (3), wobei das maschinenlesbare Erkennungsmerkmal (3) ein Merkmal des Einmalartikels (1) ist oder ein an einem vorbestimmten Teilabschnitt (11) des Einmalartikels (1) lösbar oder unlösbar, insbesondere werkseitig, angebracht ist, und
wobei der Einmalartikel (1) und/oder die medizinische Vorrichtung (2) und/oder das maschinenlesbare Erkennungsmerkmal (3), bevorzugt ein Einmalartikel (1) und/oder eine medizinische Vorrichtung (2) und/oder ein maschinenlesbares Erkennungsmerkmal (3) nach einem der vorhergehenden Ansprüche ist.

15. System nach Anspruch 14, wobei die Steuereinheit dafür vorgesehen und eingerichtet ist, anhand des detektierten und damit identifizierten Einmalartikels (1)
- eine Medikamentenauswahlmöglichkeit in der Medikamentendatenbank zu filtern und/oder zu begrenzen
- und/oder eine Auswahlmöglichkeit eines weiteren Einmalartikels (1) zu filtern und/oder zu begrenzen
- und/oder bei einer Einstellung einer Therapie der medizinischen Vorrichtung mit vorbestimmter Kombination von Medikament oder Medikamentenkonzentration zu Einmalartikel (1), einen Alarm auszugeben und/oder einen Start der Therapie zu unterbinden.

16. Ein Verfahren zur Erkennungsmerkmal abhängigen, insbesondere personalisierten, Medikamentenverabreichung in einem medizinischen System (4), insbesondere für Spritzenpumpen, umfassend:
- ein Bereitstellen einer medizinischen Vorrichtung (2), bevorzugt einer Vorrichtung (2) und/oder eines Systems nach einem der vorstehenden Ansprüche,
- ein Einbringen eines Einmalartikels (1) für die medizinische Vorrichtung (2), bevorzugt ein Einmalartikel (1) nach einem der vorstehenden Ansprüche, umfassend ein Maschinenlesbares Erkennungsmerkmal (3), wobei das maschinenlesbare Erkennungsmerkmal (3) ein Merkmal des Einmalartikels (1) ist oder ein an einem vorbestimmten Teilabschnitt (11) des Einmalartikels (1) lösbar oder unlösbar, insbesondere werkseitig, angebracht ist, und
- ein Erkennen des Einmalartikels (1), bevorzugt mittels eine Erkennungseinrichtung (23) der medizinischen Vorrichtung (2), mittels des Erkennungsmerkmals (3) des Einmalartikels (1);
- ein Identifizieren des Erkennungsmerkmals (3) des Einmalartikels (1) auf Basis der erfassten Informationen;
- ein Steuern einer Antriebseinheit, bevorzugt mittels einer Steuereinrichtung (25) der medizinischen Vorrichtung (2), zur automatisierten Verabreichung eines Medikaments gemäß erkannten Informationen des Einmalartikels (1).
